# FASCICULE DE BREVET EUROPEEN

(11) **EP 1 466 541 B1**
(45) Date de publication et mention de la délivrance du brevet: **27.02.2008**
(21) Numéro de dépôt: 04290666.9
(22) Date de dépôt: 11.03.2004
(51) Int. Cl.: A45D 40/26, A45D 2/48, A46B 11/08, H05B 3/00

(54) **Ensemble de conditionnement et d'application d'un produit de maquillage**
Vorrichtung zum Aufbewahren und Auftragen eines Schminkmittels
Device for storing and applying a make-up product

(30) Priorité: 11.04.2003 FR 0304571
(43) Date de publication de la demande: 13.10.2004
(73) Titulaire: L'ORÉAL, 75008 Paris (FR)
(72) Inventeur: De la Poterie, Valérie, 77820 Le Châtelet en Brie (FR); Marcotte, Louis, 75012 Paris (FR)
(74) Mandataire: Boulard, Denis

(56) Documents cités:
- EP-A- 0 931 476
- WO-A-01/78551
- WO-A-99/22782
- FR-A- 2 824 267
- US-A- 5 775 344
- US-A1- 2001 018 484

## Description

La présente invention a trait à un ensemble pour le conditionnement et l'application d'une composition de maquillage et/ou de soin. L'invention vise notamment le maquillage et/ou le soin des matières kératiniques, et en particulier des cils ou des sourcils.

Il existe des compositions de mascara, dites recourbantes, et destinées à être appliquées à froid.

De telles compositions, notamment à base de cires dures et polymères tenseurs, sont décrites en particulier dans le brevet EP 0 928 607. Avec ces compositions, l'effet de recourbement des cils peut être parfois jugé insuffisant. En outre, sa tenue dans le temps n'est pas optimale.

Dans la demande WO 00/74519, il est fait état de propriétés de recourbement améliorées en utilisant des organogélateurs pour remplacer tout ou partie des cires de la composition. A l'expérience, il s'avère que les résultats obtenus en termes de recourbement sont décevants.

Il existe par ailleurs différents types de dispositifs qualifiés de "recourbe-cils". Selon un premier type, le dispositif est configuré sous forme d'une pince qui, à froid, avant application du maquillage, permet de pincer les cils de manière à induire une courbure aux cils avant maquillage de ces derniers. La mise en oeuvre de cette méthode est des plus délicates à réaliser. Les résultats sont peu satisfaisants en termes d'amplitude de recourbement et de durabilité. Il est connu également d'utiliser un tel dispositif après application du produit. A l'expérience, il s'avère que la qualité du dépôt est détériorée par cette étape postérieure de mise en forme des cils.

Selon un second type, le dispositif est configuré sous forme d'une pince ou d'une brosse, et est chauffant. Le recourbement des cils avec un tel dispositif, en combinaison avec une composition de mascara classique, se fait soit préalablement au maquillage, auquel cas, l'effet de recourbement est peu perceptible après application du produit de maquillage, soit postérieurement au maquillage, auquel cas, une partie du dépôt de mascara peut être éliminé ou altéré. Des dispositifs de ce type sont décrits notamment dans la demande de brevet EP-A1-0 848 920 ou WO 99/22782 ou dans les brevets US 5 853 010 ou US 6 009 884.

Le brevet US-A-5 775 344 décrit un ensemble de conditionnement et d'application d'un produit, notamment pour les cils, comprenant un récipient contenant la composition à appliquer. Un capot destiné à obturer une ouverture du récipient est solidaire d'une tige chauffante dont une extrémité est solidaire d'un applicateur, notamment sous forme d'une brosse.

Les parois du récipient sont équipées de moyens de chauffage de manière à, préalablement à l'ouverture du récipient, en chauffer son contenu. Après extraction de l'applicateur, la chaleur émise par la tige maintient la composition présente sur l'applicateur à une température adéquate.

Selon ce document, les moyens de chauffage de la tige sont constitués d'un film chauffant s'étendant sur toute la longueur de la tige, y compris sur sa portion disposée en regard de l'essoreur. Il en résulte que ce dernier doit être réalisé en un matériau choisi de manière à résister à une élévation de température sensible, lequel matériau, du fait de ses nécessaires propriétés de résistance à la température, n'est pas forcément optimal en termes de propriétés d'essuyage de la tige.

En outre, l'intégralité de la tige étant chauffée, le système présente une inertie importante, ce qui d'un point de vue pratique, n'est pas optimal. Du fait de cette inertie importante, les moyens de chauffage équipant la tige ne peuvent au mieux qu'assurer le maintien à température de la composition qui a été chauffée par d'autres moyens. En l'occurrence, selon ce document, les parois du récipient sont elles mêmes équipées d'une feuille chauffante destinée à chauffer la composition préalablement à son utilisation. Ce sont ces moyens de chauffage qui, selon ce document, constituent les moyens de chauffage principaux du dispositif.

Le document est par ailleurs totalement silencieux sur les propriétés, notamment thermiques, de la composition à appliquer au moyen d'un tel dispositif.

Enfin, récemment, ont été commercialisés des mascaras vendus en combinaison avec un recourbe-cils du type de ceux décrits précédemment. Outre les inconvénients mentionnés ci-avant, les compositions ne sont pas stables thermiquement, et augmentent fortement en viscosité ou "prennent en masse" après un nombre répété de cycles de chauffage. En outre, le figeage des cils dans leur position recourbée ne se fait pas de manière rapide, ce qui rend l'utilisation du système des plus fastidieuses. De tels kits (recourbe-cils + mascara) ont été vendus en particulier au Japon, sous la marque commerciale Lash finity curler® avec des mascaras portant la marque Max Factor 2000 calories® N° 3, ou Stretch N° 2®.

Aussi, est-ce un des objets de l'invention que de réaliser un ensemble d'application d'un produit de maquillage et/ou de soin, notamment des cils ou des sourcils, qui résolve en tout ou partie les problèmes discutés ci-avant en référence aux systèmes de l'art antérieur.

C'est en particulier un des objets de l'invention que de réaliser un ensemble d'application d'un produit de maquillage et/ou de soin des fibres kératiniques, notamment des cils, qui permette d'obtenir un effet, notamment de recourbement ou d'allongement des fibres, qui soit à la fois notable et durable dans le temps.

C'est un autre objet de l'invention que de réaliser un ensemble de conditionnement et d'application d'un produit de maquillage et/ou de soin des matières kératiniques, destiné à être chauffé, et dont les propriétés cosmétiques ne soient pas altérées de manière sensible au fil des utilisations.

C'est encore un autre objet de la présente invention que de réaliser un ensemble qui soit de mise en oeuvre plus simple relativement à certains dispositifs conventionnels.

D'autres objets encore apparaîtront dans la description détaillée qui suit.

Selon l'invention, ces objets sont atteints en réalisant un ensemble de conditionnement et d'application d'une composition de maquillage et/ou de soin, notamment des cils ou des sourcils, comprenant :
i) un réservoir ;
ii) une composition de maquillage et/ou de soin disposée à l'intérieur du réservoir, ladite composition comportant un profil thermique ayant au moins un pic de fusion dont la largeur à mi-hauteur est inférieure ou égale à 10 °C ;
iii) un dispositif pour l'application de la composition de maquillage et/ou de soin ; et
iv) des moyens de chauffage pour porter ladite composition, préalablement, simultanément, ou postérieurement à son application, à une température supérieure à son point de fusion, et de préférence, supérieure ou égale à sa température de fin de fusion.

Ainsi, selon l'invention la combinaison d'une composition de maquillage et/ou de soin ayant un profil thermique particulier, en combinaison avec un outil permettant préalablement, simultanément, ou postérieurement à son application, de la chauffer, s'est avérée produire un effet, notamment de recourbement dans le cas des cils, qui soit à la fois d'amplitude satisfaisante, et qui soit en outre durable dans le temps.

Les compositions ayant de telles caractéristiques présentent la particularité, lorsqu'elles sont chauffées à une température supérieure à leur point de fusion Pf, et de préférence supérieure ou égale à leur température de fin de fusion, de passer d'un état amorphe (température supérieure à Pf) à un état cristallin (température inférieure à Pf) en un temps très court.

De préférence, les moyens de chauffage sont aptes à porter le produit à appliquer, à une température comprise entre 35 et 100 °C, et de préférence, entre 40 °C et 80 °C.

La très faible largeur du pic de fusion est tout particulièrement avantageuse en ce qu'elle permet lors du refroidissement du produit, de figer les cils en position recourbée, et ce, en quelques secondes.

Par "pic de fusion" on entend le pic présenté par le profil thermique de la composition obtenu par DSC, le point de fusion Pf étant la température mesurée au sommet du pic de fusion.

Le point de fusion de la composition peut être mesuré à l'aide d'un calorimètre à balayage différentiel (D.S.C.), par exemple le calorimètre vendu sous la dénomination DSC 30 par la société METLER. Un échantillon de 5 à 10 mg de produit disposé dans un creuset est soumis à une première montée en température allant de -20 °C à 90 °C, à la vitesse de chauffe de 5 °C/ minute, puis est refroidi de 90 °C à -20 °C à une vitesse de refroidissement de 5 °C/minute et enfin soumis à une deuxième montée en température allant de -20 °C à 90 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Avantageusement, le point de fusion de la composition Pf est compris entre 20 °C et 80 °C, de préférence, entre 25°C et 75 °C, et mieux encore, entre 35 °C et 60 °C.

Selon un premier mode de réalisation, les moyens de chauffage sont formés par un dispositif distinct du dispositif d'application, ledit ensemble étant configuré sous forme d'un kit. Un tel kit peut être conditionné à l'intérieur d'un conditionnement de type blister pack. Les moyens de chauffage peuvent être du type de ceux décrits dans les brevets US 6 009 884 ou US 5 853 010. D'autres dispositifs configurés sous forme d'une pince chauffante (dans le cas des cils) peuvent également être utilisés. De tels dispositifs sont décrits notamment dans le brevet US 6 220 252.

Un avantage d'une telle configuration tient au fait que le produit de maquillage peut être conditionné dans un ensemble de conditionnement et d'application classique, commercialisé :
i) soit en combinaison avec un dispositif de chauffage quand il est destiné à être utilisé selon l'invention ;
ii) soit seul quand il est destiné à être utilisé à froid.

De préférence, en fonction des caractéristiques d'application recherchées, l'utilisatrice aura la possibilité soit d'utiliser le produit à froid, soit de le chauffer pour obtenir un recourbement à la fois plus important et plus durable.

Selon une alternative, les moyens de chauffage sont associés au dispositif d'application. Ainsi, sont réduits de manière sensible l'encombrement ainsi que le coût global du dispositif. En outre, cette configuration offre la possibilité avec le même outil d'appliquer le produit à chaud, le cas échéant, et de recourber les cils en même temps, d'un seul geste. Le temps global nécessaire au maquillage s'en trouve réduit de manière substantielle.

Alternativement encore, et à la manière de ce qui est décrit dans le brevet US-A-5 775 344, les moyens de chauffage peuvent être associés au réservoir lui même, notamment à des parois intérieures de ce dernier.

De préférence, le dispositif d'application comporte un applicateur disposé au bout d'une tige et qui, en position montée du dispositif d'application sur l'ensemble, est au contact de la composition à l'intérieur du réservoir, les moyens de chauffage étant agencés de manière à ne pas chauffer de manière sensible au moins une partie de la tige. L'inertie du système s'en trouve réduite d'autant.

De préférence, la partie de la tige qui n'est pas chauffée est à une température maximale inférieure à la température de fin de fusion Tf du pic de fusion, et de préférence encore, inférieure ou égale à la température du point de fusion Pf de la composition.

De préférence, ladite partie qui n'est pas chauffée de manière sensible est, préalablement à la première utilisation de l'ensemble, apte à être disposée au dessus de la composition à l'intérieur du réservoir. C'est typiquement le cas lorsque l'ensemble est en position sensiblement verticale, en position tête en haut.

Ainsi, les portions de tige qui ne sont pas au contact du produit à réchauffer ne sont pas chauffées de manière sensible. Il n'y a donc pas d'énergie utilisée inutilement.

Par "température de fin de fusion du pic" on entend la température à laquelle 95 % de l'enthalpie de fusion est consommée.

Par "température de début de fusion du pic" on entend la température à laquelle 5 % de l'enthalpie de fusion est consommée.

Ainsi, on limite le nombre d'organes constitutifs du dispositif qui sont soumis à des changements de température importants. C'est le cas notamment de l'essoreur qui, dans une configuration telle que décrite dans le brevet US 5 775 344, est soumis à d'importantes variations de température, ce qui oblige à choisir pour sa réalisation des matériaux résistants à la chaleur, et qui n'offrent pas forcément le meilleur comportement en termes d'essorage de l'applicateur.

De préférence, les moyens de chauffage sont reliés à une source d'alimentation, de préférence en courant continu, en particulier sous forme d'une batterie, notamment rechargeable. L'alimentation peut être en particulier en 6 ou en 12 V.

La puissance dissipée par les moyens de chauffage peut être comprise entre 0,5 et 4 Watt, et de préférence, entre 0,5 et 2 Watt, et de préférence encore, entre 0,5 et 1 Watt.

Avantageusement, les moyens de chauffage comprennent une résistance chauffante configurée notamment sous forme d'un enroulement à plusieurs spires.

En particulier lorsque l'enroulement fait office d'applicateur, la forme de sa section transversale peut être choisie de manière adéquate, en fonction notamment des caractéristiques recherchées à l'application. En particulier, la section transversale de l'enroulement peut être de forme circulaire, carrée, triangulaire, hexagonale, ou autre.

La résistance chauffante comprend de préférence un fil en matériau électriquement conducteur, notamment en acier inoxydable à chaud, entouré d'un matériau électriquement isolant et thermiquement conducteur, notamment en magnésie ou en alumine.

Avantageusement, la résistance chauffante comporte un revêtement de protection extérieur, notamment en acier inoxydable à chaud.

Le diamètre maximal de la résistance chauffante peut être compris entre 0,3 et 1 mm, et de préférence, entre 0,4 et 0,6 mm.

L'enroulement peut être à spires jointives ou non jointives. Cette caractéristique est tout particulièrement importante quand l'enroulement formé par la résistance est en outre utilisé comme moyen d'application du produit. En effet, la disposition relative des spires, et en particulier leur densité, a un impact sur les caractéristiques d'application du produit de maquillage.

Comme indiqué précédemment, les moyens de chauffage peuvent faire office de moyens d'application.

Alternativement, les moyens d'application sont distincts des moyens de chauffage. Dans cette configuration, l'applicateur peut être décalé axialement sur sensiblement toute sa longueur relativement aux moyens de chauffage. Ainsi, les moyens de chauffage peuvent s'étendre, sensiblement d'une extrémité de la tige adjacente à l'applicateur à une portion de la tige située sensiblement au niveau ou en dessous du niveau de la surface libre du produit préalablement à la première utilisation de l'ensemble. Ainsi, on optimise la quantité de produit se trouvant au contact direct des moyens de chauffage, diminuant ainsi le temps nécessaire pour porter la composition à la température requise. En revanche, l'essoreur n'est pas soumis à l'élévation de température.

De préférence toutefois, il y a un recouvrement axial au moins partiel entre les moyens de chauffage et l'applicateur. Ainsi, au moins une partie du produit présent sur l'applicateur peur être chauffé simultanément à son application sur les cils. Une telle configuration offre l'avantage de donner plus de temps à l'utilisatrice pour travailler le dépôt de produit avant qu'il ne revienne à l'état cristallin.

De préférence, les moyens de chauffage s'étendent sur sensiblement toute la longueur de l'applicateur. L'homogénéité du maquillage s'en trouve améliorée. De préférence encore, ils ne s'étendent pas de manière sensible sur la tige.

L'applicateur peut comprendre un arrangement de poils maintenus entre les deux branches d'un fil torsadé, notamment d'un fil de fer. Les fibres seront réalisées en un matériau choisi pour résister aux températures auxquelles elles seront soumises. On utilise typiquement certains polyamides.

Les poils de l'applicateur peuvent être configurés sous forme d'une succession de spires imbriquées au moins en partie dans les spires de l'enroulement formé par la résistance chauffante. Ainsi, peut être utilisée une brosse torsadée classique sur laquelle on vient "visser" l'enroulement hélicoïdal formé par la résistance chauffante. La résistance chauffante est suffisamment petite pour ne pas gêner l'application du produit par les poils.

Alternativement, l'applicateur comprend un élément dont au moins une portion de la surface comporte des reliefs, notamment sous forme de stries, destinés à l'application du produit et/ou à la séparation des cils, les moyens de chauffage s'étendant sur au moins une partie de la longueur de l'applicateur, notamment à la surface de ce dernier.

Alternativement encore, l'applicateur est constitué d'un élément creux à l'intérieur duquel sont disposés les moyens de chauffage. Les moyens de chauffage peuvent être sous différentes formes. Il peut s'agir notamment d'une résistance à fil vif ou isolé, d'une lampe à incandescence, d'un gaz comprimé, d'un fluide caloporteur, en circulation ou par accumulation, d'un élément en rotation ou animé d'un mouvement alternatif pour échauffer l'applicateur par frottement mécanique, d'un élément à résistance lamellaire tamisant la surface intérieure de l'élément creux, ou de tout autre dispositif, fonctionnant notamment sur le principe d'une pompe à chaleur.

L'élément creux peut être en métal inoxydable, en plastique, ou en tout autre matériau ayant de bonnes propriétés de conduction de la chaleur. Sa surface externe peut comprendre toutes sortes d'éléments ou de reliefs (dents, poils, stries, etc.) pour favoriser l'application du produit et la séparation des cils. En particulier, une ou plusieurs rangées de poils ou de dents, s'étendant longitudinalement à l'applicateur, peuvent être prévues à la surface de l'élément creux. Ces rangées de poils ou de dents peuvent être obtenues de moulage avec l'élément creux, ou rapportées, notamment par collage.

A titre d'exemple spécifique l'élément creux est en aluminium et est de section sensiblement triangulaire avec des angles aplatis. En chacun des angles s'étend une rainure longitudinale dans laquelle est disposée une rangée de poils en polyamide.

En particulier dans cette configuration comportant un élément creux, l'applicateur peut être conçu de manière à être facilement démontable, pour le remplacer ou le nettoyer.

Notamment pour des applications autres que les cils ou les sourcils, l'applicateur peut être de configuration différente de celles qui viennent d'être exposées ci-avant. Il peut s'agir notamment d'un pinceau, d'un bloc de mousse à cellules ouvertes, semi-ouvertes ou fermées, d'un fritté, ou de toute autre structure choisie en fonction de la nature et de la localisation de la surface à traiter et/ou à maquiller.

Bien entendu, les moyens de chauffage peuvent être associés à des moyens de contrôle, notamment sous forme de moyens de mesure de la température et/ou de moyens indicateurs de température. Ces moyens de contrôle peuvent permettre notamment, de :
i) limiter la température (bi-lame) ; et/ou
ii) indiquer la température (éléments thermochromes) ; et/ou
iii) mesurer et réguler la température (thermocouple).

Une extrémité de la tige opposée à l'applicateur se termine de préférence par un capot obturant une ouverture du réservoir lorsque l'applicateur est disposé à l'intérieur du réservoir.

De préférence, un essoreur est disposé au voisinage de ladite ouverture, et destiné à être traversé par l'applicateur, lors de son extraction du réservoir. Un tel essoreur peut être réalisé en polyéthylène ou en matériau élastomérique. Comme indiqué précédemment, les moyens de chauffage sont agencés avantageusement de sorte qu'une portion de la tige située en regard de l'essoreur, lorsque le capot obture ladite ouverture, ne puisse pas être chauffée de manière sensible par les moyens de chauffage.

De préférence, la composition de maquillage et/ou de soin est stable thermiquement. En d'autres termes, sa consistance n'est pas altérée de manière sensible par les changements de température subis à chaque utilisation. En raison de cette caractéristique, les propriétés cosmétiques de la composition ne sont pas altérées de manière sensible entre le première utilisation et la dernière.

Par composition "stable thermiquement" on désigne une composition dont la viscosité varie d'au plus 25 % et de préférence, d'au plus 20 %, et de préférence encore, d'au plus 15 %, et de préférence encore, d'au plus 10 %, après avoir été soumise à une succession de X cycles de fusion/refroidissement selon le protocole suivant : On dispose la composition dans une étuve à 80 °C, pendant 2 heures. On laisse ensuite la composition revenir naturellement à température ambiante. On en mesure la viscosité après mise en oeuvre des X cycles. Entre deux cycles successifs, on laisse 24h. La viscosité mesurée après mise en oeuvre des X cycles de fusion/refroidissement est comparée avec celle mesurée avant le premier cycle.

De préférence, X = 4 . De préférence encore, X = 8. De préférence encore X = 10. De préférence encore, X = 15.

De préférence, les mesures de viscosité sont faites au Rhéomat RM 180 équipé d'un mobile MS-r3 ou Ms-r4 tournant à 240 min⁻¹ pour une alimentation en courant à 60 Hz ou à 200 min⁻¹ pour une alimentation en courant à 50 Hz.

Cette caractéristique est particulièrement avantageuse lorsque le chauffage de la composition a lieu à l'intérieur du récipient la contenant. Cette caractéristique est également avantageuse du fait des résidus de produit subsistant inévitablement sur l'applicateur après chaque application.

De préférence, le profil thermique de la composition de maquillage présente un pic de fusion, situé de préférence dans une plage de température comprise entre 10 °C et 90 °C. On peut observer sur le profil la présence de "pics" supplémentaires qui, généralement, sont d'amplitude sensiblement plus faible et de largeur plus importante.

Avantageusement, la température de début de fusion T0 est supérieure ou égale à 10 °C, de préférence, supérieure ou égale à 15 °C, et de préférence encore, supérieure ou égale à 20 °C. De préférence T0 est comprise entre 10 °C et 50 °C, de préférence, entre 15 °C et 45 °C, et de préférence encore entre 20 °C et 40 °C.

De préférence également, la température de fin de fusion Tf est inférieure ou égale à 90 °C, et de préférence, inférieure ou égale à 80 °C, et de préférence encore, inférieure ou égale à 70 °C, et de préférence encore, inférieure ou égale à 60 °C. De préférence, Tf est comprise entre 35 °C et 90 °C, de préférence, entre 35 °C et 80 °C, de préférence encore, entre 40 °C et 70 °C, et de préférence encore, entre 40 °C et 60 °C.

L'amplitude de température du pic de fusion (ΔT=Tf - T0) est comprise entre 1 °C et 30 °C, et de préférence entre 2 °C et 25 °C, et de préférence encore, entre 3°C et 20 °C.

De préférence également, la largeur du pic de fusion Lf à mi-hauteur est comprise entre 1 °C et 10 °C, de préférence, entre 1,5 °C et 8 °C, et de préférence encore, entre 2 °C et 5 °C. La mi-hauteur du pic de fusion peut être déterminée sur la base de la demi distance entre une droite reliant deux portions planes du profil thermique de part et d'autre du pic de fusion, et le sommet du pic.

La composition selon l'invention peut être notamment à phase continue, anhydre ou aqueuse, en émulsion ou en dispersion.

De préférence, la composition de maquillage comprend une phase grasse contenant au moins un composé choisi parmi les cires, les polymères semi-cristallins, les huiles, les huiles épaissies par un agent structurant et leurs mélanges.

De préférence encore, la phase grasse comprend un polymère semi-cristallin ayant un point de fusion supérieur à 20 °C. De tels polymères semi-cristallins sont notamment décrits dans la demande de brevet FR-A-2 824 267.

Par "polymère semi-cristallin", on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.

La phase grasse de la composition peut comprendre en outre une cire qui, de préférence, est en mélange minoritaire avec un polymère semi-cristallin. Dans la présente demande, une cire est un composé lipophile, solide à température ambiante (25 °C), à changement d'état solide/liquide réversible, ayant un point de fusion supérieur ou égal à 30 °C pouvant aller jusqu'à 120 °C. En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

De préférence, la cire est choisie parmi la cire d'olive obtenue par hydrogénation d'huile d'olive estérifiée avec l'alcool stéarylique telle que la Phytowax Olive 18L57, l'alcool stéarique, le stéarate de stéaryle, le benzoate de stéaryle, le ditriméthylolpropanetetrastéarate, le beurre de palme, la cire Licowax KST de chez Clariant, le ditrimethylolpropanetetrabehenate, ou la cire de dioctadecylcarbonate et leurs mélanges

La phase grasse peut également comprendre une huile choisie parmi les huiles hydrocarbonées et/ou siliconées et/ou fluorées volatiles ou non volatiles et leurs mélanges.

La phase grasse peut également comprendre une huile épaissie par un agent structurant. L'agent structurant peut être choisi parmi les gélifiants lipophiles, les organogélateurs et leurs mélanges.

La composition peut en outre comprendre une phase aqueuse qui peut être constituée essentiellement d'eau. La phase aqueuse peut comporter un agent épaississant.

La composition peut également comprendre une phase anhydre constituée essentiellement de solvants volatiles, notamment siliconés, hydrocarbonés, ou fluorés.

La composition peut également comprendre une matière colorante comme les matières colorantes pulvérulentes, notamment des pigments, les colorants liposolubles, les colorants hydrosolubles.

La composition peut comprendre des polymères filmogènes, hydrosolubles, liposolubles ou sous forme dispersée.

La composition peut comprendre, en outre, tout additif usuellement utilisé en cosmétique tels que les antioxydants, les conservateurs, les parfums, les neutralisants, les plastifiants, les actifs cosmétiques comme par exemple des émollients, des hydratants, des vitamines, des filtres solaires, et leurs mélanges. Ces additifs peuvent être présents dans la composition en une teneur allant de 0,01 à 15 %, du poids total de la composition.

Bien entendu l'homme du métier veillera à choisir les éventuels additifs complémentaires et/ou leur quantité de telle manière que les propriétés avantageuses de la composition ne soient pas altérées par l'adjonction envisagée, et notamment de manière que le profil thermique de la composition reste tel que défini plus haut.

La composition peut être fabriquée par les procédés connus, généralement utilisés dans le domaine cosmétique.

La composition utilisée selon l'invention peut être une composition de maquillage, une base de maquillage, notamment des fibres kératiniques, ou base-coat, une composition à appliquer sur un maquillage, dite encore top-coat, ou bien encore une composition de traitement des fibres kératiniques. Plus spécialement, la composition selon l'invention est un mascara.

D'autres applications toutefois peuvent être envisagées. On peut en effet citer les compositions de maquillage et/ou de soin de la peau pour lesquelles l'apport de chaleur peut être bénéfique au résultat maquillage et/ou à la facilité d'application du produit. A titre d'exemples, il peut s'agir de fond de teint, de fard à paupières, de rouge à lèvres, d'un liner, etc.

Selon un autre aspect de l'invention, on réalise également une recharge destinée, en particulier lorsque les moyens de chauffage sont intégrés au dispositif d'application, à équiper un ensemble de conditionnement et d'application selon l'invention. La recharge comprend un réservoir contenant le produit de maquillage, et dont un bord délimite une ouverture. De préférence, la recharge est équipée d'un essoreur disposé au voisinage de l'ouverture.

Avant utilisation, l'ouverture de la recharge est obturée par élément de fermeture amovible. Il peut s'agir d'un bouchon, notamment vissé, ou de tout autre moyen adéquat, notamment, un opercule thermoscellé.

L'invention consiste, mises à part les dispositions exposées ci-dessus, en un certain nombre d'autres dispositions qui seront explicitées ci-après, à propos d'exemples de réalisation non limitatifs, décrits en référence aux figures annexées, parmi lesquelles :
- la figure 1 est relative à un premier mode de réalisation de l'ensemble de conditionnement et d'application selon l'invention ;
- la figure 2 illustre le profil thermique obtenu par DSC d'un exemple de composition de maquillage utilisable selon l'invention ;
- les figures 3A et 3B sont relatives à un second mode de réalisation de l'ensemble de conditionnement et d'application selon l'invention ;
- la figure 4 illustre une première variante du mode de réalisation des figures 3A et 3B ;
- la figure 5 illustre une seconde variante du mode de réalisation des figures 3A et 3B ;
- la figure 6 illustre une troisième variante du mode de réalisation des figures 3A et 3B ;
- la figure 7 illustre une quatrième variante du mode de réalisation des figures 3Aet3B;
- la figure 8 illustre une cinquième variante du mode de réalisation des figures 3A et 3B ,
- la figure 9 illustre une sixième variante du mode de réalisation des figures 3A et 3B ; et
- la figure 10 représente une recharge destinée à être utilisée avec un ensemble de conditionnement et d'application selon l'invention.

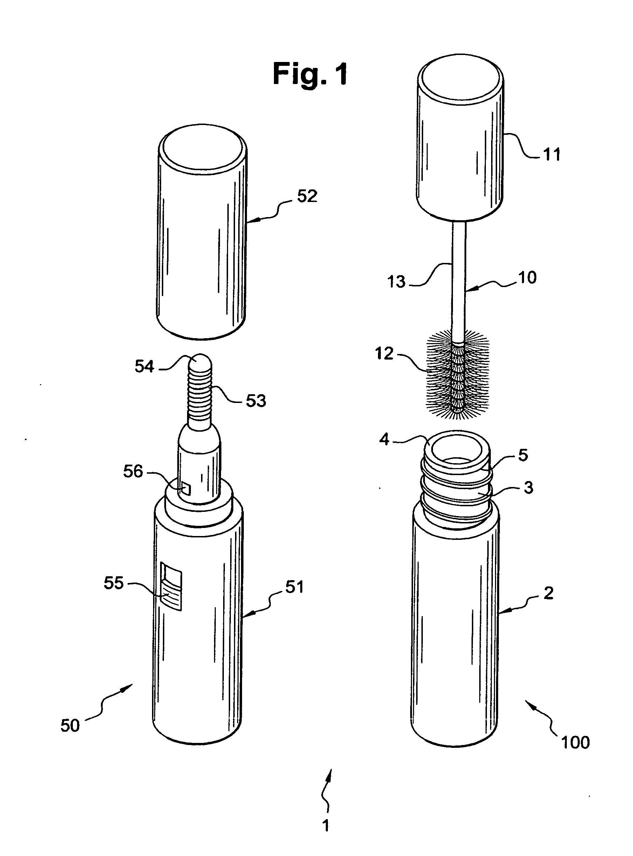

Le kit 1 décrit à la figure 1 comprend un ensemble de conditionnement et d'application 100 du mascara et un dispositif de chauffage 50, séparé de l'ensemble de conditionnement et d'application.

Les deux dispositifs 100 et 50 peuvent être vendus ensemble dans un même conditionnement, de type blister pack. L'unité 100 contenant le produit peut être vendue séparément.

L'ensemble de conditionnement et d'application 100 comprend un récipient 2 surmonté d'un col fileté 3 dont un bord libre délimite une ouverture 4. Dans l'ouverture 4, est monté un organe d'essorage 5. L'ensemble 100 comprend également un dispositif d'application 10 comprenant un bouchon 11 solidaire d'une tige 13 dont une extrémité comporte un applicateur 12, configuré généralement sous forme d'un arrangement de fibres maintenues entre les deux branches d'un fil de fer torsadé. Une surface intérieure du bouchon 11 est filetée de manière à coopérer avec le filetage du col 3. Ainsi, lorsque l'applicateur 12 et la tige 13 sont disposés à l'intérieur du récipient 2, le filetage du bouchon 11 vient en engagement avec le filetage du col 3 de manière à ce que le bouchon obture de manière étanche l'ouverture 4 du récipient. De tels ensembles de conditionnement et d'application sont bien connus.

La composition contenue à l'intérieur du récipient 2 est la suivante :
- Polymère semi-cristallin (polyacrylate de stéaryle) 23,3 g
- Polybutène 11,7 g
- Acide stéarique 5,8 g
- Aminopropanediol 0,5 g
- Hydroxyéthylcellulose 0,9 g
- Gomme arabique 3,45 g
- Triéthanolamine 2,4 g
- Pigments 8 g
- Conservateurs qs
- Eau qsp 100 g

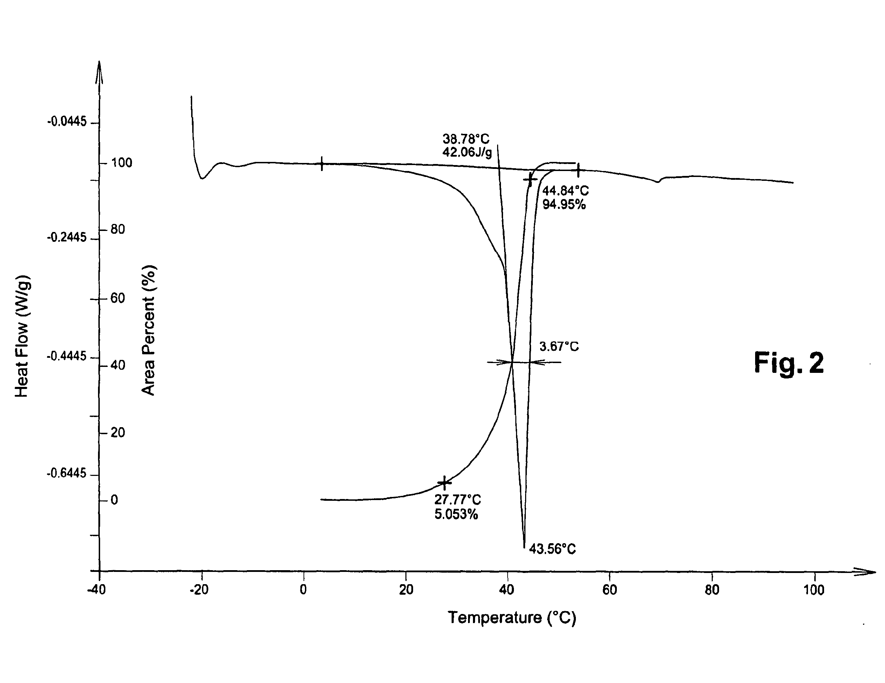

Son profil thermique est représenté à la figure 2. Comme il apparaît sur la figure 2, le profil thermique présente un seul pic de fusion. La température de début de fusion T0 est de 27,77 °C. La température de fin de fusion Tf est de 44,84 °C. L'amplitude de température ΔT est de 17,07 °C. La largeur du pic de fusion à mi-hauteur Lf est de 3,67 °C. Le point de fusion Pf de la composition est de 43,56 °C.

Après 15 cycles de fusion/refroidissement selon le protocole décrit ci-avant, la variation de viscosité de la composition est de 5,9 %.

Le dispositif de chauffage 50 est conforme à ce qui est décrit dans le brevet US 6 009 884. Il comprend principalement une partie de préhension 51 et un capuchon 52. Une batterie est disposée à l'intérieur de la partie de préhension 51, et est connectée à un fil chauffant 53 configuré sous forme d'un enroulement hélicoïdal disposé sur une tige 54. Un "switch" 55 permet de mettre en tension, respectivement d'éteindre le dispositif. Une LED 56, lorsqu'elle change de couleur, indique que le dispositif est à la température requise, et qu'il est donc prêt à être utilisé.

L'alimentation de la partie chauffante via la batterie est en 12 V. La puissance dissipée est d'environ 1 Watt.

Le fil chauffant 53 peut entre réalisé en un alliage nickel/chrome.

Selon ce mode de réalisation, le mascara est appliqué à froid de manière classique sur les cils au moyen de la brosse 12. Le mouvement de recourbement des cils peut être imprimé à ces derniers lors de l'application du produit à froid ou préalablement à son application au moyen d'un dispositif chauffant.

Après application à froid du produit, l'utilisatrice met en engagement la partie chauffante 53 du dispositif 50 avec les cils de manière à porter le dépôt de produit à une température supérieure à la température de fin de fusion de la composition, tout en imprimant aux cils un mouvement visant à les recourber. Typiquement, la composition est portée à environ 50 - 70 °C. A cette température, la composition est dans un état amorphe. En refroidissant, la composition revient à son état cristallin, et ceci de manière très rapide en raison de la faible largeur du pic de fusion. Les cils sont figés dans leur configuration recourbée recherchée et ceci de façon durable.

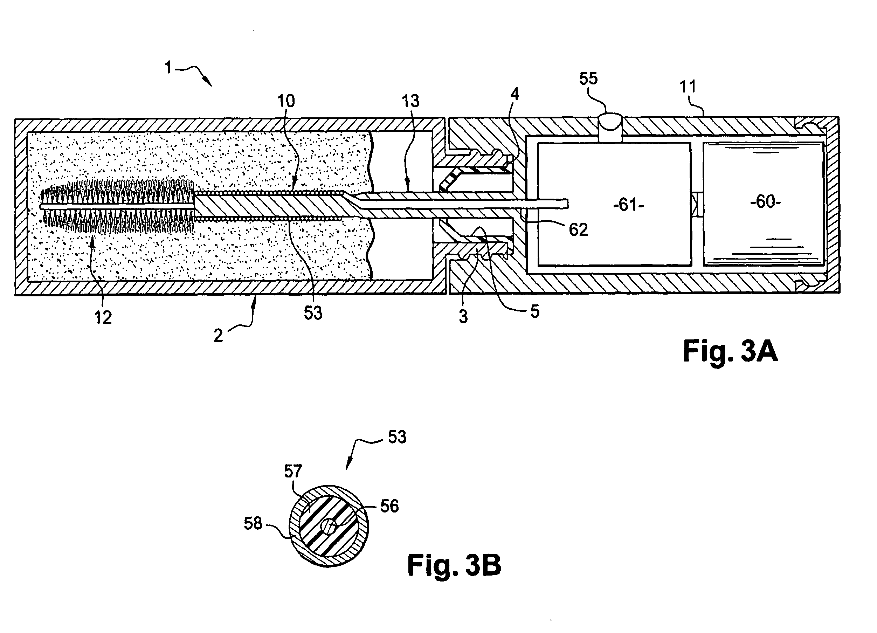

Dans le mode de réalisation des figures 3A et 3B auxquelles il est maintenant fait référence, les moyens de chauffage sont directement associés au moyens d'application du produit.

Ainsi, l'ensemble de conditionnement et d'application 1 selon ce mode de réalisation comprend un récipient 2 conforme au récipient 2 de la figure 1.

A l'intérieur du capuchon 11 est disposé une source d'alimentation en courant continu 60, notamment sous forme d'une batterie rechargeable, reliée à une électronique de régulation 61. Un interrupteur sous forme d'un poussoir 55 permet sélectivement d'activer/désactiver la partie chauffante 53 du dispositif d'application 10. Une LED est intégrée au poussoir 55 et change de couleur lorsque la composition contenue dans le réservoir 2 est à la température adéquate.

Tout comme dans le mode de réalisation précédent le capuchon 11 est solidaire d'une tige 13 à extrémité de laquelle est fixée une brosse 12 de type brosse torsadée.

L'électronique de régulation est reliée à une résistance 53 configurée sous forme d'un fil enroulé sur la surface extérieure de la tige 13. L'enroulement est à spires jointives et s'étend sensiblement de l'extrémité de la tige adjacente à la brosse 12, à une portion de la tige située sensiblement au niveau de la surface libre du produit à l'intérieur du récipient préalablement à la première utilisation du dispositif. Comme nous le verrons plus en détail par la suite, la partie de la tige située au dessus de l'enroulement hélicoïdal n'est pas chauffée de manière sensible. C'est notamment le cas de la portion de la tige située en regard de la lèvre de l'organe d'essorage 5.

Comme il apparaît sur la vue en coupe de la figure 3B, la résistance chauffante 53 comprend un coeur 56 constitué d'un fil en acier inoxydable à chaud d'un diamètre d'environ 0,1 mm. Le fil 56 est entouré d'un isolant électrique 57, bon conducteur de la chaleur. Typiquement, il s'agit de magnésie ou d'alumine. Le tout est disposé à l'intérieur d'une gaine en acier inoxydable 58. Le diamètre extérieur de la résistance est d'environ 0,5 mm. Une telle résistance chauffante est vendue notamment par la société THERMOCOAX sous la référence 2NcNcAc.

Entre la partie enroulée sur la tige 13 (résistance 53) et le circuit de régulation 61, les fils 62 passent à l'intérieur de la tige 13. Leur diamètre (de l'ordre du mm) est sensiblement supérieur à celui de la résistance chauffante 53, de manière à ce qu'il ne soient pas chauffés de manière sensible sur cette portion. On réduit ainsi au maximum l'inertie du système. De même, du fait de l'absence sensible de chauffage de la tige en regard de l'essoreur, ce dernier peut être réalisé en matériau conventionnel.

La composition contenue à l'intérieur du récipient est identique à celle du mode de réalisation précédent :

Ainsi, selon ce mode de réalisation, l'utilisatrice au moyen de l'interrupteur 55 active le chauffage de la résistance 53. Cette dernière est au contact du produit à l'intérieur du récipient 2 et le porte à une température supérieure à sa température de fin de fusion. Typiquement, la composition est chauffée à environ 60°.

Lorsque la température est atteinte, la LED de l'interrupteur 55 passe au vert. L'utilisatrice dévisse alors le capuchon 11, et extrait l'applicateur du récipient 2.

Lors du mouvement d'extraction, la brosse 12 est essorée de manière conventionnelle. Le produit ainsi chauffé est alors appliqué de manière conventionnelle, en imprimant aux cils un mouvement destiné à les recourber.

En refroidissant, la composition revient à son état cristallin, et ceci de manière très rapide en raison de la faible largeur du pic de fusion. Les cils sont figés dans leur configuration recourbée recherchée et ceci de façon durable.

La composition utilisée selon l'invention présente une très bonne stabilité thermique. En effet, sa consistance n'est pas altérée par les changements de température subis à chaque cycle de chauffage/refroidissement. De ce fait, ses propriétés cosmétiques ne sont pas altérées de manière sensible au fil des utilisations.

Le dispositif selon ce mode de réalisation est particulièrement avantageux relativement au précédent en ce que, avec un seul passage, au moyen d'un seul et même outil, le produit est appliqué et les cils recourbés de manière durable.

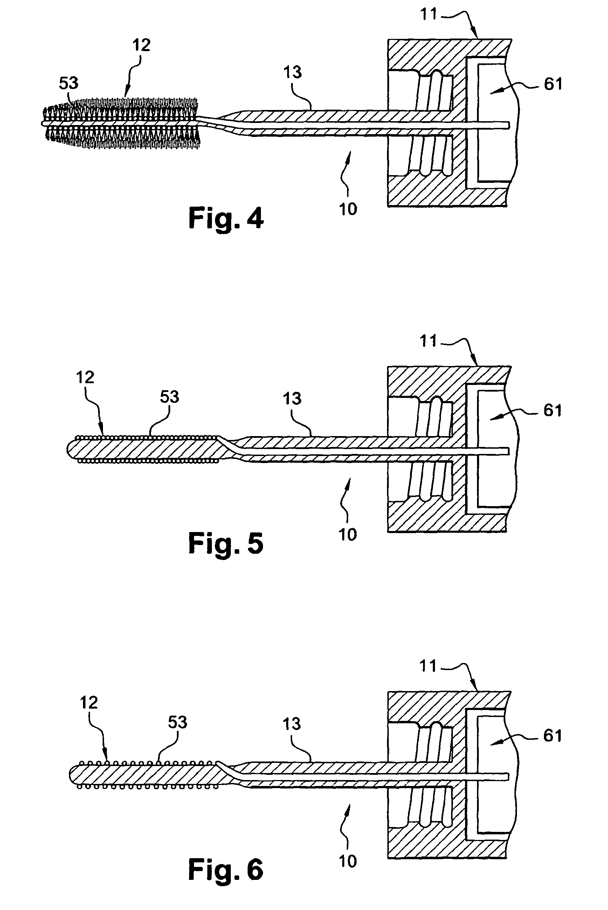

Dans la variante de la figure 4, les spires de l'enroulement formé par la résistance 53 sont imbriquées dans les spires formées par l'arrangement de poils de la brosse 12, la résistance chauffante 53 s'étendant sensiblement sur toute la longueur de la brosse 12.

La composition de mascara est identique à celle du mode de réalisation précédent.

Le fonctionnement du dispositif selon ce mode de réalisation est identique à celui du mode de réalisation précédent, un avantage majeur résidant dans le fait que la composition sur la brosse continue d'être chauffée lors de son application sur les cils. L'utilisatrice dispose donc de plus de temps pour travailler son dépôt, notamment pour conférer aux cils le recourbement souhaité.

Dans la variante de la figure 5, les moyens d'application du produit sont constitués directement par les spires de l'enroulement formé par la résistance chauffante 53. Ce mode de réalisation est de conception sensiblement plus économique que le mode de réalisation précédent.

Selon ce mode de réalisation de la figure 5, les spires de l'enroulement 53 sont sensiblement jointives. Dans une autre variante illustrée à la figure 6, les spires sont écartées. Le choix de telle configuration est dicté en particulier par les caractéristiques recherchées pour le dépôt de mascara sur les cils.

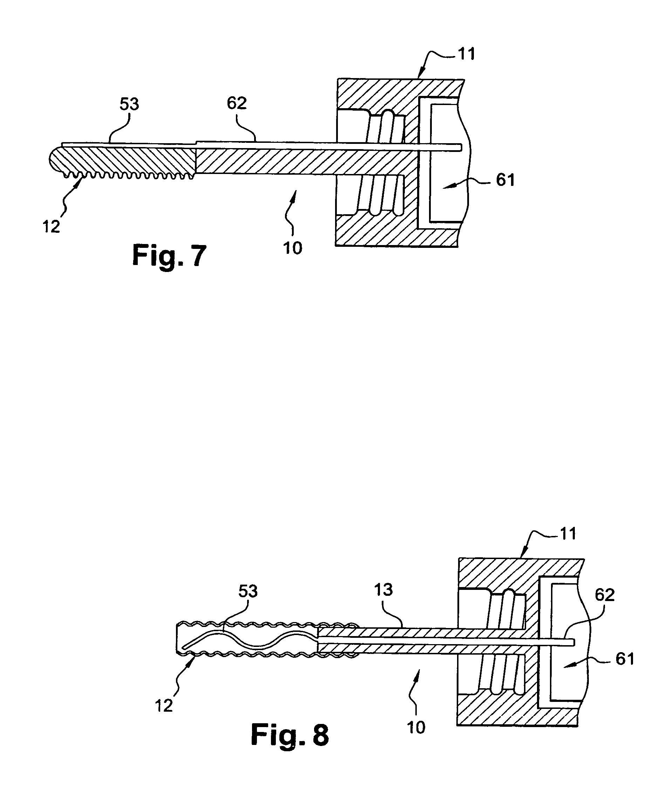

Dans le mode de réalisation de la figure 7, l'applicateur 12 est constitué d'un cylindre métallique dont au moins une partie de sa périphérie, est striée perpendiculairement à son axe longitudinal. Le cylindre strié est fixé, notamment par collage, au bout de la tige 13. De manière diamétralement opposée relativement à la partie striée, est disposée une résistance chauffante 53, s'étendant sur sensiblement toute la longueur de l'applicateur 12. La résistance chauffante 53 peut être disposée dans une rainure ménagée longitudinalement dans la surface du cylindre.

Sur la partie d'application 12, le diamètre de la résistance chauffante 53 est d'environ 0,5 mm. Les portions 62 reliant le circuit d'alimentation 61 à la résistance chauffante sont d'un diamètre d'environ 1 mm.

Ainsi, la résistance chauffante 53 chauffe la composition présente sur le cylindre strié, la zone striée de ce dernier servant à l'application proprement dit du produit sur les cils, ainsi qu'à leur séparation.

Dans le mode de réalisation de la figure 8, l'applicateur 12 est constitué d'un cylindre creux, notamment en acier inoxydable, et monté serrant sur l'extrémité de la tige 13. L'élément chauffant 53 est constitué d'une résistance électrique, à fil vif, et s'étendant à l'intérieur du cylindre creux sur sensiblement toute sa longueur. Pour faciliter le chauffage du cylindre en inox, la résistance 53 est configurée en hélice de manière à être sensiblement au contact de la surface interne du cylindre creux.

Comme pour les autres modes de réalisation, sur la portion 62 reliant l'alimentation à la partie chauffante 53, les fils sont de diamètre plus important de manière à ce que la tige 13 ne soit pas chauffée de manière sensible. Seul l'applicateur 12 est chauffé.

La surface extérieure du cylindre est striée de manière à faciliter l'application du produit et la séparation des cils.

Cette configuration est avantageuse en ce que l'élément chauffant 53 est ni visible ni accessible. Il n'y a pas de contact direct avec le produit. La température de l'applicateur 12 est homogène.

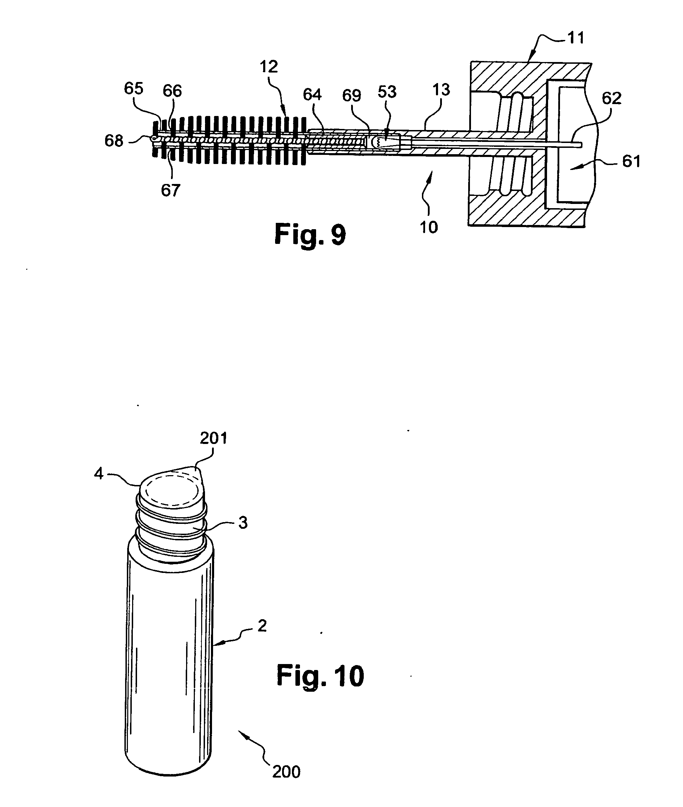

Dans le mode de réalisation de la figure 9, la tige 13 se termine par une partie creuse 69 au fond de laquelle est disposée une lampe à incandescence 53 alimentée en courant continu (5V-115 mA) via le circuit 62. La puissance de la lampe est de 575 mW. A titre d'exemple, on utilise une lampe commercialisée par la société ORBITEC® sous la référence OR 7153.

A l'intérieur de la partie creuse 69 de la tige 13 est engagé à force un élément tubulaire 65 en métal inoxydable, et dont une extrémité se prolonge bien au delà de la partie creuse 69 de la tige. Au moins sur sa portion située à l'extérieur de la tige 13, la paroi de l'élément tubulaire 65 est traversée par deux fentes 66, 67, s'étendant de manière hélicoïdale (décalées de 180°).

A l'intérieur de l'élément tubulaire 65, au voisinage de son extrémité située à l'intérieur de la tige 13, est disposé un insert tubulaire 64 en matériau résistant à la chaleur, et destiné à recevoir de façon légèrement serrante l'extrémité non empoilée de l'âme torsadée 68 d'une brosse 12. La partie empoilée de la brosse comprend un arrangement de poils configuré sous forme d'une double hélice. Le pas de chacune des hélices est sensiblement identique au pas des fentes 66 et 67 de sorte que, lorsque la brosse est insérée par vissage dans l'élément tubulaire 65, les spires de l'arrangement de poils de la brosse soient situées à l'intérieur des fentes 66 et 67 et que les poils de la brosse émergent à l'extérieur de l'élément tubulaire 65.

L'extrémité non empoilée de l'âme torsadée 68 de la brosse 12 est engagée de façon serrante à l'intérieur de l'insert tubulaire 64.

Ainsi, selon ce mode de réalisation, la lampe à incandescence 53 génère, au voisinage d'une extrémité de l'élément 65, de la chaleur qui est transmise par conduction jusqu'à l'autre extrémité de ce dernier, permettant ainsi au produit prélevé par les poils de la brosse 12 d'être chauffé de manière adéquate. Comme pour les autres modes de réalisation, la partie de la tige 13 adjacente au capuchon 11 n'est pas chauffée de manière sensible.

Un applicateur de ce type s'utilise à la manière de n'importe quelle brosse torsadée, le chauffage du produit se faisant soit à l'intérieur du récipient, préalablement à l'extraction de l'applicateur 10, soit pendant l'application, soit après l'application. Alternativement aussi, un tel applicateur 10 peut être utilisé sans faire usage de la chaleur.

Selon une variante de ce mode de réalisation, l'élément tubulaire 65 est traversé par une ou plusieurs fentes s'étendant de manière longitudinale et au travers desquelles émergent une ou plusieurs rangées longitudinales de poils de la brosse 12 disposée à l'intérieur de l'élément tubulaire. De préférence, les fentes sont au nombre de trois, espacées à 120°.

La figure 10 à laquelle il est maintenant fait référence représente une recharge 200 destinée à remplacer, notamment après utilisation totale de son contenu, le récipient 2 d'un ensemble de conditionnement et d'application 1 tel que discuté en référence aux figures 3A et 3B. La recharge 200 comprend un réservoir 2 contenant le produit de maquillage, et dont un bord délimite une ouverture 4. La recharge 200 est équipée d'un essoreur disposé au voisinage de l'ouverture 4.

Le filetage du col 3 de la recharge est adapté au filetage formé par le bouchon du dispositif d'application chauffant 10.

Avant utilisation, l'ouverture 4 de la recharge 200 est obturée par élément de fermeture 201 amovible constitué d'un opercule thermoscellé.

Dans la description détaillée qui précède, il a été fait référence à des modes de réalisation préférés de l'invention.

## Revendications

1. - Ensemble de conditionnement et d'application (1) d'une composition de maquillage des cils ou des sourcils, comprenant :
i) un réservoir (2) ;
ii) une composition de maquillage des cils ou des sourcils disposée à l'intérieur du réservoir ;
iii) un dispositif (10) pour l'application de la composition de maquillage et
iv) des moyens de chauffage (53) pour porter ladite composition, préalablement, simultanément, ou postérieurement à son application, à une température supérieure à son point de fusion, et de préférence, supérieure ou égale à sa température de fin de fusion,
**caractérisé en ce que** ladite composition comporte un profil thermique ayant un pic de fusion dont la largeur à mi-hauteur Lf est inférieure ou égale à 10 °C.

2. - Ensemble de conditionnement et d'application (1) selon la revendication 1 **caractérisé en ce que** les moyens de chauffage (53) sont formés par un dispositif (50) distinct du dispositif d'application (10), ledit ensemble étant configuré notamment sous forme d'un kit.

3. - Ensemble de conditionnement et d'application (1) selon la revendication 1 **caractérisé en ce que** les moyens de chauffage (53) sont associés au dispositif d'application (10) et/ou au réservoir (2).

4. - Ensemble de conditionnement et d'application (1) selon la revendication 3 **caractérisé en ce que** le dispositif d'application (10) comporte un applicateur (12) disposé au bout d'une tige (13) et qui, en position montée du dispositif d'application (10) sur le réservoir (2), est au contact de la composition à l'intérieur du réservoir, les moyens de chauffage (53) étant agencés de manière à ne pas chauffer de manière sensible au moins une partie de la tige (13) qui, préalablement à la première utilisation de l'ensemble, est apte à être disposée au dessus de la composition à l'intérieur du réservoir (2).

5. - Ensemble de conditionnement et d'application (1) selon l'une quelconque des revendications 1 à 4 **caractérisé en ce que** les moyens de chauffage (53) sont reliés à une source d'alimentation (60), de préférence en courant continu, en particulier sous forme d'une batterie, notamment rechargeable.

6. - Ensemble (1) selon l'une quelconque des revendications 1 à 5 **caractérisé en ce que** la puissance dissipée par les moyens de chauffage (53) est comprise entre 0,5 et 4 Watt, et de préférence, entre 0,5 et 2 Watt, et de préférence encore, entre 0,5 et 1 Watt.

7. - Ensemble (1) selon l'une quelconque des revendications 1 à 6 **caractérisé en ce que** les moyens de chauffage (53) comprennent une résistance chauffante configurée notamment sous forme d'un enroulement à plusieurs spires.

8. - Ensemble (1) selon la revendication 7 **caractérisé en ce que** la résistance chauffante (53) comprend un fil (56) en matériau électriquement conducteur, notamment en acier inoxydable à chaud, entouré d'un matériau électriquement isolant et thermiquement conducteur (57), notamment en magnésie ou en alumine.

9. - Ensemble (1) selon la revendication 8 **caractérisé en ce que** la résistance chauffante (53) comporte un revêtement de protection extérieur (58), notamment en acier inoxydable à chaud.

10. - Ensemble (1) selon l'une quelconque des revendications 7 à 9 **caractérisé en ce que** la résistance chauffante (53) à un diamètre compris entre 0,3 et 1 mm, et de préférence, entre 0,4 et 0,6 mm.

11. - Ensemble (1) selon l'une quelconque des revendications 7 à 10 **caractérisé en ce que** l'enroulement est à spires jointives.

12. - Ensemble (1) selon l'une quelconque des revendications 7 à 10 **caractérisé en ce que** l'enroulement est à spires non jointives.

13. - Ensemble (1) selon la revendication 4 **caractérisé en ce qu'**il y a un recouvrement axial au moins partiel entre les moyens de chauffage (53) et l'applicateur (12).

14. - Ensemble (1) selon la revendication 13 **caractérisé en ce que** les moyens de chauffage (53) s'étendent sur sensiblement toute la longueur de l'applicateur (12).

15. - Ensemble (1) selon la revendication 3 **caractérisé en ce que** les moyens de chauffage (53) font office d'applicateur (12).

16. - Ensemble (1) selon la revendication 3 **caractérisé en ce que** l'applicateur (12) est distinct des moyens de chauffage (53).

17. - Ensemble (1) selon la revendication 3 **caractérisé en ce que** l'applicateur (12) est décalé axialement sur sensiblement toute sa longueur relativement aux moyens de chauffage (53).

18. - Ensemble (1) selon la revendication 17 **caractérisé en ce que** les moyens de chauffage (53) s'étendent sensiblement d'une extrémité de la tige (13) adjacente à l'applicateur (12) à une portion de la tige située sensiblement au niveau ou en dessous du niveau de la surface libre du produit préalablement à la première utilisation de l'ensemble, lorsque le réservoir (2) est en position sensiblement verticale .

19. - Ensemble (1 ) selon l'une quelconque des revendications précédentes **caractérisé en ce que** l'applicateur (12) comprend un arrangement de poils maintenus entre les deux branches d'un fil torsadé, notamment d'un fil de fer.

20. - Ensemble (1) selon les revendications 7 et 19 **caractérisé en ce que** les poils sont configurés sous forme d'une succession de spires imbriquées au moins en partie dans les spires de l'enroulement formé par la résistance chauffante (53).

21. - Ensemble (1) selon la revendication 7 **caractérisé en ce que** l'applicateur (12) comprend un élément dont au moins une portion de la surface comporte des reliefs, notamment sous forme de stries, destinés à l'application du produit et/ou, le cas échéant, à la séparation des cils, les moyens de chauffage (53) s'étendant sur au moins une partie de la longueur de l'applicateur (12), notamment à la surface de ce dernier.

22. - Ensemble (1) selon la revendication 3 **caractérisé en ce que** l'applicateur comprend un élément creux à l'intérieur duquel sont disposés les moyens de chauffage (53), ces derniers étant notamment sous forme d'une lampe à incandescence.

23. - Ensemble (1) selon la revendication 4 **caractérisé en ce qu**'une extrémité de la tige (13) opposée à l'applicateur (12) se termine par un capot (11) obturant une ouverture (4) du réservoir (2) lorsque l'applicateur (12) est disposé à l'intérieur du réservoir.

24. - Ensemble (1) selon la revendication 23 **caractérisé en ce qu**'il comprend un essoreur (5) disposé au voisinage de ladite ouverture, et destiné à être traversé par l'applicateur (12), lors de son extraction du réservoir (2).

25. - Ensemble (1) selon les revendications 23 et 24 **caractérisé en ce que** les moyens de chauffage (53) sont agencés de sorte qu'une portion de la tige (13) située en regard de l'essoreur (5), lorsque le capot (11) obture ladite ouverture, ne puisse pas être chauffée de manière sensible.

26. - Ensemble (1) selon l'une quelconque des revendications qui précèdent
**caractérisé en ce que** la composition de maquillage et/ou de soin est stable thermiquement.

27. - Ensemble (1) selon l'une quelconque des revendications précédentes **caractérisé en ce que** le profil thermique de la composition présente un pic de fusion, situé de préférence dans une plage de température comprise entre 10 °C et 90 °C.

28. - Ensemble (1) selon l'une quelconque des revendications précédentes **caractérisé en ce que** la température de début de fusion T0 est supérieure ou égale à 10 °C, et de préférence, supérieure ou égale à 15 °C, et de préférence encore, supérieure ou égale à 20 °C.

29. - Ensemble (1) selon l'une quelconque des revendications précédentes **caractérisé en ce que** la température de fin de fusion Tf est inférieure ou égale à 90 °C, et de préférence, inférieure ou égale à 80 °C, et de préférence encore, inférieure ou égale à 70 °C, et de préférence encore, inférieure ou égale à 60 °C.

30. - Ensemble (1) selon l'une quelconque des revendications précédentes **caractérisé en ce que** l'amplitude de température du pic de fusion (ΔT) est comprise entre 1 °C et 30 °C, et de préférence entre 2 °C et 25 °C, et de préférence encore, entre 3°C et 20 °C.

31. - Ensemble (1) selon l'une quelconque des revendications précédentes **caractérisé en ce que** la largeur du pic de fusion à mi-hauteur Lf est comprise entre 1 °C et 10 °C, et de préférence, entre 1,5 °C et 8 °C, et de préférence encore, entre 2 °C et 5 °C.

32. - Ensemble (1) selon l'une des revendications précédentes, **caractérisé en ce que** la composition comprend une phase grasse contenant au moins un composé choisi parmi les cires, les polymères semi-cristallins, les huiles, les huiles épaissies par un agent structurant et leurs mélanges.

33. - Ensemble (1) selon la revendication précédente, **caractérisé en ce** la phase grasse comprend un polymère semi-cristallin ayant un point de fusion supérieur à 20°C.

34. - Recharge (200) destinée à équiper un ensemble de conditionnement et d'application (1) selon l'une quelconque des revendications 3 ainsi que toutes celles qui en dépendent, ladite recharge comprenant un réservoir (2) contenant un produit de maquillage des cils ou des sourcils, et dont un bord délimite une ouverture (4) obturée par un élément de fermeture amovible, notamment un bouchon ou un opercule thermoscellé (201),
**caractérisée en ce que** ledit produit comporte un profil thermique ayant un pic de fusion dont la largeur à mi-hauteur Lf est inférieure ou égale à 10°C.

## Claims

1. Assembly (1) for storing and applying an eyelash or eyebrow make-up composition, comprising:
i) a container (2);
ii) an eyelash or eyebrow make-up composition placed inside the container;
iii) a device (10) for applying the make-up composition; and ,'
iv) heating means (53) for bringing said composition, prior to, at the same time as, or after its application, to a temperature above its melting point, and preferably a temperature greater than or equal to its end-of-melting temperature,
**characterized in that** said composition comprises a temperature profile having a melting peak the width at half-height Wₘ of which is less than or equal to 10°C.

2. Storage and application assembly (1) according to Claim 1, **characterized in that** the heating means (53) are formed by a device (50) separate from the application device (10), said assembly being especially configured in the form of a kit.

3. Storage and application assembly (1) according to Claim 1, **characterized in that** the heating means (53) are combined with the application device (10) and/or the container (2).

4. Storage and application assembly (1) according to Claim 3, **characterized in that** the application device (10) comprises an applicator (12) placed at the end of a wand (13) and which, in the assembled position of the application device (10) in the container (2), is in contact with the composition inside the container, the heating means (53) being arranged so as not to substantially heat at least one part of the wand (13) which, prior to the first use of the assembly, is able to be positioned at the top of the composition inside the container (2).

5. Storage and application assembly (1) according to any one of Claims 1 to 4, **characterized in that** the heating means (53) are connected to a power supply (60), preferably a DC power supply, in particular in the form of a battery, especially a rechargeable battery.

6. Assembly (1) according to any one of Claims 1 to 5, **characterized in that** the power dissipated by the heating means (53) is between 0.5 and 4 W, and preferably between 0.5 and 2 W, and more preferably between 0.5 and 1 W.

7. Assembly (1) according to any one of Claims 1 to 6, **characterized in that** the heating means (53) comprise a heating resistor configured in particular in the form of a winding having several turns.

8. Assembly (1) according to Claim 7, **characterized in that** the heating resistor (53) comprises a wire (56) made of an electrically conductive material, especially high-temperature stainless steel, surrounded by an electrically insulating and thermally conductive material (57), especially made of magnesia or alumina.

9. Assembly (1) according to Claim 8, **characterized in that** the heating resistor (53) comprises an outer protective coating (58), especially made of high-temperature stainless steel.

10. Assembly (1) according to any one of Claims 7 to 9, **characterized in that** the heating resistor (53) has a diameter between 0.3 and 1 mm, and preferably between 0.4 and 0.6 mm.

11. Assembly (1) according to any one of Claims 7 to 10, **characterized in that** the winding has contiguous turns.

12. Assembly (1) according to any one of Claims 7 to 10, **characterized in that** the winding has non-contiguous turns.

13. Assembly (1) according to Claim 4, **characterized in that** there is an at least partial axial overlap between the heating means (53) and the applicator (12).

14. Assembly (1) according to Claim 13, **characterized in that** the heating means (53) extend over approximately the entire length of the applicator (12).

15. Assembly (1) according to Claim 3, **characterized in that** the heating means (53) act as the applicator (12).

16. Assembly (1) according to Claim 3, **characterized in that** the applicator (12) is distinct from the heating means (53).

17. Assembly (1) according to Claim 3, **characterized in that** the applicator (12) is axially offset over approximately its entire length relative to the heating means (53).

18. Assembly (1) according to Claim 17, **characterized in that** the heating means (53) extend approximately from one end of the wand (13) adjacent to the applicator (12) to a portion of the wand located approximately at the level or below the level of the free surface of the product prior to the first use of the assembly, when the container (2) is in an approximately vertical position.

19. Assembly (1.) according to any one of the preceding claims, **characterized in that** the applicator (12) comprises an arrangement of bristles held between the two arms of a twisted wire, especially an iron wire.

20. Assembly (1) according to Claims 7 and 19, **characterized in that** the bristles are configured in the form of a succession of turns at least partly interleaved in the turns of the winding formed by the heating resistor (53).

21. Assembly (1) according to Claim 7, **characterized in that** the applicator (12) comprises an element of which at least one portion of the surface comprises reliefs, especially in the form of ridges, intended for applying the product and/or, where appropriate, separating the eyelashes, the heating means (53) extending over at least part of the length of the applicator (12), especially at the surface of the latter.

22. Assembly (1) according to Claim 3, **characterized in that** the applicator comprises a hollow element inside which the heating means (53) are arranged, these heating means especially being in the form of an incandescent lamp.

23. Assembly (1) according to Claim 4, **characterized in that** one end of the wand (13) opposite the applicator (12) ends in a cap (11) sealing an opening (4) of the container (2) when the applicator (12) is placed inside the container.

24. Assembly (1) according to Claim 23, **characterized in that** it comprises a wiper (5) placed in the vicinity of said opening, and intended to be passed through by the applicator (12), during its removal from the container (2).

25. Assembly (1) according to Claims 23 and 24, **characterized in that** the heating means (53) are arranged so that a portion of the wand (13) located opposite the wiper (5), when the cap (11) seals said opening, cannot be substantially heated.

26. Assembly (1) according to any one of the preceding claims, **characterized in that** the make-up and/or care composition is thermally stable.

27. Assembly (1) according to any one of the preceding claims, **characterized in that** the temperature profile of the composition has a melting peak, preferably located in a temperature range between 10°C and 90°C.

28. Assembly (1) according to any one of the preceding claims, **characterized in that** the start-of-melting temperature T₀ is greater than or equal to 10°C, preferably greater than or equal to 15°C, and more preferably greater than or equal to 20°C.

29. Assembly (1) according to any one of the preceding claims, **characterized in that** the end-of-melting temperature T_{f} is less than or equal to 90°C, and preferably less than or equal to 80°C and more preferably less than or equal to 70°C and more preferably less than or equal to 60°C.

30. Assembly (1) according to any one of the preceding claims, **characterized in that** the temperature range of the melting peak (ΔT) is between 1°C and 30°C, preferably between 2°C and 25°C and more preferably between 3°C and 20°C.

31. Assembly (1) according to any one of the preceding claims, **characterized in that** the width of the melting peak at half-height Wₘ is between 1°C and 10°C, preferably between 1.5°C and 8°C and more preferably between 2°C and 5°C.

32. Assembly (1) according to one of the preceding claims, **characterized in that** the composition comprises a fatty phase containing at least one compound chosen from waxes, semicrystalline polymers, oils, oils thickened by a structuring agent and mixtures thereof.

33. Assembly (1) according to the preceding claim, **characterized in that** the fatty phase comprises a semicrystalline polymer having a melting point above 20°C.

34. Refill (200) intended to equip a storage and application assembly (1) according to any one of Claim 3 and all the claims which depend thereon, said refill comprising a container (2) containing an eyelash or eyebrow make-up product and of which one edge delimits an opening (4) sealed by a removable closure element, especially a heat-sealed cover or lid (201), **characterized in that** said product comprises a temperature profile having a melting peak the width at half-height Wₘ of which is less than or equal to 10°C.

## Patentansprüche

1. Einheit (1) zur Verpackung und zum Auftragen einer Schminkzusammensetzung für die Wimpern oder die Augenbrauen, die aufweist:
i) ein Reservoir (2);
ii) eine Schminkzusammensetzung für die Wimpern oder die Augenbrauen, die im Inneren des Reservoirs angeordnet ist;
iii) eine Vorrichtung (10) zum Auftragen der Schminkzusammensetzung; und
iv) Heizmittel (53), um die Zusammensetzung vorher, gleichzeitig mit oder nach ihrem Auftragen auf eine Temperatur zu bringen, die höher ist als ihr Schmelzpunkt, und vorzugsweise höher als die oder gleich ihrer Temperatur am Ende des Schmelzvorgangs,
**dadurch gekennzeichnet, dass** die Zusammensetzung ein thermisches Profil aufweist, das eine Schmelzspitze hat, deren Breite auf halber Höhe Lf geringer als oder gleich 10°C ist.

2. Einheit (1) zur Verpackung und zum Auftragen nach Anspruch 1, **dadurch gekennzeichnet, dass** die Heizmittel (53) von einer Vorrichtung (50) gebildet werden, die von der Auftragvorrichtung (10) getrennt ist, wobei die Einheit insbesondere in Form eines Sets konfiguriert ist.

3. Einheit (1) zur Verpackung und zum Auftragen nach Anspruch 1, **dadurch gekennzeichnet, dass** die Heizmittel (53) der Auftragvorrichtung (10) und/oder dem Reservoir (2) zugeordnet sind.

4. Einheit (1) zur Verpackung und zum Auftragen nach Anspruch 3, **dadurch gekennzeichnet, dass** die Auftragvorrichtung (10) einen Applikator (12) aufweist, der am Ende einer Stange (13) angeordnet ist und der in der auf das Reservoir (2) montierten Stellung der Auftragvorrichtung (10) mit der Zusammensetzung im Inneren des Reservoirs n Kontakt ist, wobei die Heizmittel (53) so angeordnet sind, dass sie mindestens einen Teil der Stange (13) nicht wesentlich erwärmen, die vor der ersten Benutzung der Einheit über der Zusammensetzung im Inneren des Reservoirs (2) angeordnet werden kann.

5. Einheit (1) zur Verpackung und zum Auftragen nach einem der Ansprüche 1 bis 4, **dadurch gekennzeichnet, dass** die Heizmittel (53) mit einer Quelle (60) zur Versorgung vorzugsweise mit Gleichstrom, speziell in Form einer insbesondere wiederaufladbaren Batterie, verbunden sind.

6. Einheit (1) nach einem der Ansprüche 1 bis 5, **dadurch gekennzeichnet, dass** die von den Heizmitteln (53) abgeführte Leistung zwischen 0,5 und 4 Watt, und vorzugsweise zwischen 0,5 und 2 Watt, und noch vorzugsweise zwischen 0,5 und 1 Watt liegt.

7. Einheit (1) nach einem der Ansprüche 1 bis 6, **dadurch gekennzeichnet, dass** die Heizmittel (53) einen Heizwiderstand aufweisen, der insbesondere in Form einer Wicklung mit mehreren Windungen konfiguriert ist.

8. Einheit (1) nach Anspruch 7, **dadurch gekennzeichnet, dass** der Heizwiderstand (53) einen Draht (56) aus elektrisch leitendem Material, insbesondere aus rostfreiem Warmarbeitsstahl aufweist, der von einem elektrisch isolierenden und wärmeleitenden Material (57), insbesondere aus Magnesiumoxid oder aus Aluminiumoxid, umgeben ist.

9. Einheit (1) nach Anspruch 8, **dadurch gekennzeichnet, dass** der Heizwiderstand (53) eine äußere Schutzverkleidung (58), insbesondere aus rostfreiem Warmarbeitsstahl, aufweist.

10. Einheit (1) nach einem der Ansprüche 7 bis 9, **dadurch gekennzeichnet, dass** der Heizwiderstand (53) einen Durchmesser zwischen 0,3 und 1 mm, und vorzugsweise zwischen 0,4 und 0,6 mm, aufweist.

11. Einheit (1) nach einem der Ansprüche 7 bis 10, **dadurch gekennzeichnet, dass** die Wicklung aneinandergrenzende Windungen aufweist.

12. Einheit (1) nach einem der Ansprüche 7 bis 10, **dadurch gekennzeichnet, dass** die Wicklung nicht aneinandergrenzende Windungen aufweist.

13. Einheit (1) nach Anspruch 4, **dadurch gekennzeichnet, dass** es eine zumindest teilweise axiale Überdeckung zwischen den Heizmitteln (53) und dem Applikator (12) gibt.

14. Einheit (1) nach Anspruch 13, **dadurch gekennzeichnet, dass** die Heizmittel (53) sich über im Wesentlichen die ganze Länge des Applikators (12) erstrecken.

15. Einheit (1) nach Anspruch 3, **dadurch gekennzeichnet, dass** die Heizmittel (53) als Applikator (12) dienen.

16. Einheit (1) nach Anspruch 3, **dadurch gekennzeichnet, dass** der Applikator (12) von den Heizmitteln (53) getrennt ist.

17. Einheit (1) nach Anspruch 3, **dadurch gekennzeichnet, dass** der Applikator (12) axial über im Wesentlichen seine ganze Länge bezüglich der Heizmittel (53) versetzt ist.

18. Einheit (1) nach Anspruch 17, **dadurch gekennzeichnet, dass** die Heizmittel (53) sich im Wesentlichen von einem dem Applikator (12) benachbarten Ende der Stange (13) bis zu einem Abschnitt der Stange erstrecken, der sich im Wesentlichen in Höhe von oder unter der Höhe der freien Fläche des Produkts vor der ersten Benutzung der Einheit befindet, wenn das Reservoir (2) in im Wesentlichen senkrechter Stellung ist.

19. Einheit (1) nach einem der vorhergehenden Ansprüche, **dadurch gekennzeichnet, dass** der Applikator (12) eine Anordnung von Borsten aufweist, die zwischen den zwei Zweigen eines gewundenen Drahts, insbesondere eines Eisendrahts, gehalten werden.

20. Einheit (1) nach den Ansprüche 7 und 19, **dadurch gekennzeichnet, dass** die Borsten in Form einer Folge von Windungen konfiguriert sind, die zumindest zum Teil in die Windungen der vom Heizwiderstand (53) geformten Wicklung eingefügt sind.

21. Einheit (1) nach Anspruch 7, **dadurch gekennzeichnet, dass** der Applikator (12) ein Element aufweist, von dem zumindest ein Abschnitt der Oberfläche Reliefs, insbesondere in Form von Riefen, aufweist, die zum Auftragen des Produkts und/oder ggf. zur Trennung der Wimpern bestimmt sind, wobei die Heizmittel (53) sich über mindestens einen Teil der Länge des Applikators (12), insbesondere an dessen Oberfläche, erstrecken.

22. Einheit (1) nach Anspruch 3, **dadurch gekennzeichnet, dass** der Applikator ein hohles Element aufweist, in dessen Innerem die Heizmittel (53) angeordnet sind, wobei letztere insbesondere die Form einer Glühlampe haben.

23. Einheit (1) nach Anspruch 4, **dadurch gekennzeichnet, dass** ein Ende der Stange (13) entgegengesetzt zum Applikator (12) in einer Kappe (11) endet, die eine Öffnung (4) des Reservoirs (2) verschließt, wenn der Applikator (12) im Inneren des Reservoirs angeordnet ist.

24. Einheit (1) nach Anspruch 23, **dadurch gekennzeichnet, dass** sie einen Abstreifer (5) aufweist, der in der Nähe der Öffnung angeordnet und dazu bestimmt ist, vom Applikator (12) bei seinem Herausziehen aus dem Reservoir (2) durchquert zu werden.

25. Einheit (1) nach den Ansprüchen 23 und 24, **dadurch gekennzeichnet, dass** die Heizmittel (53) so angeordnet sind, dass ein Abschnitt der Stange (13), der sich vor dem Abstreifer (5) befindet, wenn die Kappe (11) die Öffnung verschließt, nicht wesentlich erwärmt werden kann.

26. Einheit (1) nach einem der vorhergehenden Ansprüche, **dadurch gekennzeichnet, dass** die Schmink- und/oder Pflegezusammensetzung thermisch stabil ist.

27. Einheit (1) nach einem der vorhergehenden Ansprüche, **dadurch gekennzeichnet, dass** das thermische Profil der Zusammensetzung eine Schmelzspitze aufweist, die sich vorzugsweise in einem Temperaturbereich zwischen 10°C und 90°C befindet.

28. Einheit (1) nach einem der vorhergehenden Ansprüche, **dadurch gekennzeichnet, dass** die Schmelzanfangstemperatur T0 höher als oder gleich 10°C, und vorzugsweise höher als oder gleich 15°C, und noch vorzugsweise höher als oder gleich 20°C ist.

29. Einheit (1) nach einem der vorhergehenden Ansprüche, **dadurch gekennzeichnet, dass** die Schmelzendetemperatur Tf niedriger als oder gleich 90°C, und vorzugsweise niedriger als oder gleich 80°C, und noch vorzugsweise niedriger als oder gleich 70°C, und noch vorzugsweise niedriger als oder gleich 60°C ist.

30. Einheit (1) nach einem der vorhergehenden Ansprüche, **dadurch gekennzeichnet, dass** die Temperaturamplitude der Schmelzspitze (ΔT) zwischen 1°C und 30°C, und vorzugweise zwischen 2°C und 25°C, und noch vorzugsweise zwischen 3°C und 20°C liegt.

31. Einheit (1) nach einem der vorhergehenden Ansprüche, **dadurch gekennzeichnet, dass** die Breite der Schmelzspitze auf halber Höhe Lf zwischen 1 °C und 10°C, und vorzugsweise zwischen 1,5°C und 8°C, und noch vorzugsweise zwischen 2°C und 5°C liegt.

32. Einheit (1) nach einem der vorhergehenden Ansprüche, **dadurch gekennzeichnet, dass** die Zusammensetzung eine fette Phase aufweist, die mindestens eine Verbindung enthält, die aus den Wachsen, den halbkristallinen Polymeren, den Ölen, den durch einen strukturierenden Wirkstoff verdickten Ölen und ihren Mischungen ausgewählt wird.

33. Einheit (1) nach dem vorhergehenden Anspruch, **dadurch gekennzeichnet, dass** die fette Phase ein halbkristallines Polymer aufweist, das einen Schmelzpunkt höher als 20°C hat.

34. Nachfüllung (200), die dazu bestimmt ist, eine Einheit zur Verpackung und zum Auftragen (1) nach einem der Ansprüche 3 sowie allen davon abhängenden zu bestücken, wobei die Nachfüllung ein Reservoir (2) aufweist, das ein Schminkprodukt für die Wimpern oder die Augenbrauen enthält und von dem ein Rand eine Öffnung (4) begrenzt, die von einem entfernbaren Verschlusselement bedeckt wird, insbesondere einem Stopfen oder einer wärmeversiegelten Abdeckfolie (201), **dadurch gekennzeichnet, dass** das Produkt ein thermisches Profil aufweist, das eine Schmelzspitze hat, deren Breite Lf auf halber Höhe geringer als oder gleich 10°C ist.
